(19) Europäisches Patentamt
European Patent Office
Office européen des brevets

(11) Publication number: **0 330 463 A1**

(12) **EUROPEAN PATENT APPLICATION**

(21) Application number: **89301758.2**

(22) Date of filing: **23.02.89**

(51) Int. Cl.4: **A 61 B 5/02**
A 61 B 8/06

(30) Priority: **24.02.88 JP 41678/88**

(43) Date of publication of application:
**30.08.89 Bulletin 89/35**

(84) Designated Contracting States:
**DE FR GB IT NL SE**

(71) Applicant: **COLIN ELECTRONICS CO., LTD.**
**2007-1, Hayashi**
**Komaki-shi Aichi-ken (JP)**

(72) Inventor: **Ogletree, William A.**
**3421 Saw Mill Road**
**Newtown Square PA 19073 (US)**

**Kawamura, Norio**
**c/o Colin Electronics Company Ltd. 2007-1, Hayashi**
**Komaki-shi Aichi-ken (JP)**

**Yokoe, Hifumi**
**c/o Colin Electronics Company Ltd. 2007-1, Hayashi**
**Komaki-shi Aichi-ken (JP)**

(74) Representative: **Senior, Alan Murray et al**
**J.A. KEMP & CO 14 South Square Gray's Inn**
**London WC1R 5EU (GB)**

(54) **Blood flow resistance measuring apparatus.**

(57) An apparatus for detecting a peripheral resistance opposing blood flow through a peripheral portion (20) of the subject, including a first determining device (12, 36) for determining blood pressure of the subject, a second determining means (38, 40, 44, 36) for determining at least one of a flow rate and a flow amount of blood which flows through an arterial vessel (22) extending through the peripheral portion (20) of the subject; and a calculating device (36) for calculating the peripheral resistance of the subject, based on the blood pressure and the at least one of the blood flow rate and the blood flow amount.

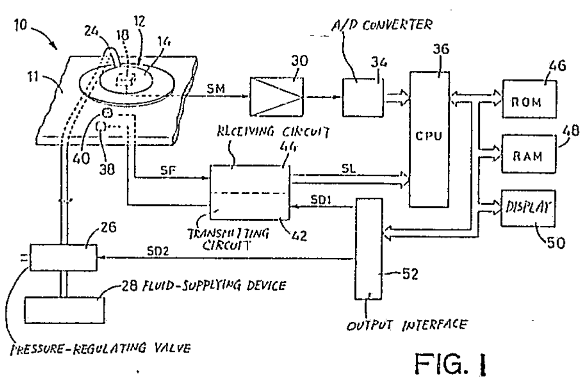


FIG. I

EP 0 330 463 A1

**Description**

## BLOOD FLOW RESISTANCE MEASURING APPARATUS

The present invention relates to an apparatus for measuring the resistance opposing blood flowing through a pheripheral portion of a subject's body.

It is widely practiced to continuously measure blood pressure of a living body, for example to monitor the circulatory organ of the living body during, or after, a surgical operation. Blood pressure can vary through various causes; for example, it is decreased as a result of a decrease in cardiac output, a decrease in resistance, a high volume of bleeding, etc. Accordingly, in addition to the blood pressure measurement, it is also possible to measure concurrently cardiac output and/or to follow the variation in resistance indirectly through measurement of skin temperature. However, measuring skin temperature can be an inaccurate way of measuring the resistance, because it varies with room temperature. Thus, it has been difficult to reliably monitor dynamic condition of the circulatory organ of a subject or rapidly detect a state of shock of a subject.

The present invention, provides an apparatus for detecting a resistance opposing blood flow through a peripheral portion of a subject's body, comprising: first determining means for determining blood pressure of the subject; a second determining means for determining at least one of a flow rate and a flow amount of blood which flows through an arterial vessel extending through the peripheral portion of said body; and calculating means for calculating the resistance based on the blood pressure and the at least one of the blood flow rate and the blood flow amount.

In the resistance measuring apparatus constructed as described above, the calculating means calculates the resistance of a peripheral portion of a subject based on the blood pressure determined by the first determining means, and the flow rate or flow amount of the blood flowing through an arterial vessel extending through the peripheral portion, the flow rate or flow amount being determined by the second determining means. Thus, the present apparatus is capable of directly measuring the resistance of the subject. Consequently, the present apparatus permits more reliable monitoring of dynamic condition of the circulatory organ of a patient and a more rapid detection of a shock of a patient.

In a preferred embodiment of the resistance measuring apparatus of the present invention, the second determining means comprises an ultrasonic wave generator for generating ultrasonic wave toward the arterial vessel extending through the peripheral portion of the subject, an ultrasonic wave detector for detecting the ultrasonic wave reflected by the blood flowing through the arterial vessel, and means for determining the blood flow rate based on the detected ultrasonic wave.

In a modified form of the above embodiment of the invention, the first determining means comprises a pressure sensor for detecting pulse wave produced from the arterial vessel extending through the peripheral portion of the subject, the pressure sensor being pressed against a body surface of the subject just above the arterial vessel so as to detect the pulse wave, and means for determining the blood pressure based on magnitude of the detected pulse wave.

According to a feature of the above-indicated modified form of the apparatus of the invention, the pressure sensor comprises an elastic member for defining a fluid-tight space, the elastic member being pressed against the arterial vessel via the body surface when pressure in the fluid-tight space is increased, and a pressure transducer disposed in the fluid-tight space, for detecting pressure variation in the fluid-tight space upon transmission of the pulse wave to the fluid-tight space from the arterial vessel.

According to another feature of the above-indicated modified form of the invention, the apparatus further comprises a wrist band for supporting, on one of opposite surfaces thereof, the ultrasonic wave generator and detector and the pressure sensor, the wrist band pressing, under the one of opposite surfaces thereof, the ultrasonic wave generator and detector and the pressure sensor against a radial artery of the subject via the body surface when the wrist band is wound around a wrist of the subject. In this case, it is recommended that the apparatus further comprise third determining means for determining oxygen saturation of blood which flows through capillaries in skin of the subject, the third determining means comprising a first light emitter for emitting toward the skin a light beam having a first wavelength, a second light emitter for emitting toward the skin a light beam having a second wavelength different from the first wavelength, and a light detector for detecting the light beams reflected from the skin, the wrist band also supporting the first and second light emitters and the light detector, the third determining means further comprising means for determining the oxygen saturation based on the detected light beams.

In another embodiment of the apparatus of the invention, the second determining means determines at least one of an average flow rate and an average flow amount of the blood flowing through the arterial vessel.

In yet another embodiment of the invention, the apparatus further comprises a display device for displaying at least one value of maximum, minimum and average blood pressure; maximum, minimum and average blood flow rate; maximum, minimum and average resistance; and maximum, minimum and average value obtained by respectively multiplying the maximum, minimum and average resistance by an average sectional area of the arterial vessel.

In a modifed form of the above embodiment of the invention, the display device displays the at least one value each time the pressure sensor detects a pulse of the pulse wave from the arterial vessel.

In a further embodiment of the apparatus of the invention, the second determining means determines the blood flow amount by multiplying the blood flow rate by an average sectional area of the arterial vessel.

The invention will be further described by way of non-limitative example, with reference to the accompanying drawings, in which:-

Fig. 1 is a diagrammatic view of a peripheral resistance measuring apparatus of the present invention;

Fig. 2 is a cross-sectional view of a pressure sensor of the apparatus of Fig. 1 supported by a wrist band wound around a wrist of a subject;

Fig. 3 is a flow chart illustrating the operation of the apparatus of Fig. 1;

Fig. 4 is a graph including curves of peripheral resistance and maximum, minimum and average blood pressure; and

Fig. 5 is a view corresponding to Fig. 1, showing another embodiment of the apparatus of the invention.

Referring to Figs. 1 and 2, there is shown a peripheral resistance measuring apparatus of the present invention.

In Fig. 1 reference numeral 10 designates a wrist band supporting on an inner surface 11 thereof a pressure sensor 12. When the wrist band 10 is set around a wrist 20 (Fig. 2) of a subject, the inner surface 11 of the band 10 is opposed to a body surface 19 of the wrist 20. The pressure sensor 20 includes an elstic hat-like member 14 formed of a thin rubber sheet, and a pressure transducer 18. The hat-like member 14 has an outer flange at which the hat-like member 14 is secured to the inner surface 11 of the wrist band 10 so as to define an air-tight space 16 in which the pressure transducer 18 is disposed. The wrist band 10 is wound around the wrist 20 such that the hat-like member 14 is aligned with a radial artery 22 extending through the wrist 20. The radial artery 22 is an arterial vessel extending near a radius of the subject.

The air-tight space 16 communicates with a fluid supplying device 28 via a piping 24 and a pressure-regulating valve 26, and is supplied with pressurized air or other sorts of pressurized fluid from the fluid supplying device 28. When pressure in the air-tight space 16 is increased by the pressurized air supplied thereto, the hat-like member 14 is pressed against the radial artery 22 via the body surface 19 of the wrist 20. The pressure transducer 18 detects, as pulse wave, pressure variation produced in the air-tight space 16 synchronously with pulsation of the radial artery 22 (i.e., heartbeat of the subject), and generates pulse wave signal SM whose magnitude corresponds to magnitude of the detected pulse wave, to a CPU (central processing unit) 36 via an amplifier 30 and an A/D (analog-to-digital) converter 34.

As shown in Fig. 1, the inner surface 11 of the wrist band 10 also supports an ultrasonic wave generator 38 and an ultrasonic wave detector 40 such that the generator 38 and the detector 40 each are located adjacent to the hat-like member 14 of the pressure sensor 12 and spaced apart by a predetermined distance from each other, and that the generator 38 is located upstream of the detector 40 along the radial artery 22 when the wrist band 10 is wound around the wrist 20. Thus, the generator and detector 38, 40 are positioned right above the radial artery 22 with the wrist band 10 set around the wrist 20. The ultrasonic wave generator 38 continuously generates ultrasonic wave having a predetermined frequency toward the radial artery 22, according to drive signal SD1 supplied thereto from an output interface 52 via a transmitting circuit 42. Meanwhile, the ultrasonic wave detector 40 detects the ultrasonic wave reflected by blood flowing through the radial artery 22, and generates frequency signal SF representing the frequency of the reflected ultrasonic wave, to a receiving circuit 44. The receiving circuit 44 includes an arithmetic circuit for detecting a frequency shift of the reflected wave, or a phase difference between the non-reflected wave generated by the generator 38 and the reflected wave detected by the detector 40, based on frequency signal SF received, determining a flow rate of the blood flowing through the radial artery 22 based on the detected frequency shift or phase difference, and generating flow rate signal SL representing the determined blood flow rate, to the CPU 36.

The CPU 36 is connected to a ROM (read only memory) 46, a RAM (random access memory) 48, a display 50 and the output interface 52. The CPU 36 processes the received signals according to programs pre-stored in the ROM 46 and by utilizing temporary-storage function of the RAM 48. The CPU 36 generates drive signal SD2 via the output interface 52 to the pressure-regulating valve 26 to regulate the pressure in the air-tight space 16 of the pressure sensor 12. The CPU 36 determines maximum, minimum and average blood pressure of the subject based on magnitude of pulse wave signal SM supplied from the pressure sensor 12, and commands the display 50 to display the determined blood pressure as shown in a lower portion of the graph of Fig. 4. Further, the CPU 36 generates drive signal SD1 via the output interface 52 to the transmitting circuit 42 to activate the ultrasonic wave generator 38 to generate the ultrasonic wave toward the radial artery 22. Moreover, the CPU 36 calculates an averate blood flow rate during a time period corresponding to each pulse of the pulse wave represented by pulse wave signal SM, based on flow rate signal SF received, determines a peripheral resistance of the subject regarding each pulse of the pulse wave based on the determined average blood pressure and the determined average blood flow rate, and commands the display 50 to time-wise display the determined peripheral resistance as shown in an upper portion of the graph of Fig. 4.

Referring next to the flow chart of Fig. 3, there will be described the operation of the present peripheral resistance measuring apparatus.

At a step (not shown) the CPU 36 commands the pressure-regulating valve 26 to regulate the pressurized fluid supplied from the fluid supplying device 28 to the air-tight space 16, so that the amplitude of pulse wave signal SM representing the pulse wave produced from the radial artery 22, is increased to a maximum. Next, the control of the CPU 36 goes to step S1 at which the CPU 36 reads in pulse wave signal SM. Step S1 is followed by step S2 at which the CPU 36 reads in flow rate signal SF. At the following step S3 it is judged whether or not signals SM and SL corresponding to one pulse of the pulse wave have been read in. If the judgement at step S3 is negative (NO), steps S1 through S3 are repeated to continue to read in signals SM and SF until the judgement at step S3 is turned to be affirmative (YES). Once the judgement at step S3 is affirmative, step S3 is followed by step S4 at which the CPU 36 determines maximum blood pressure $BP_{max}$, minimum blood pressure $BP_{min}$ and average blood pressure $BP_{av}$ based on the magnitude of the read pulse wave signal SM and according to a predetermined relationship between blood pressure and magnitude of pulse wave signal SM. In the present embodiment, the pressure sensor 12, step S4 stored in the form of program in the ROM 46, the CPU 36 and the RAM 48 for effecting step S4, and others cooperate with each other to serve as the means for determining the blood pressure of the subject.

Step S4 is followed by step S5 at which the CPU 36 calculates an average blood flow rate $V_{av}$ based on the read flow rate signal SL corresponding to one pulse of the pulse wave, and at the following step S6 the CPU 36 determines a peripheral resistance based on the average blood pressure $BP_{av}$ and the average blood flow rate $V_{av}$.

Peripheral resistance R is expressed by the following equation (1):

$R = BP_{av} / ( V_{av} \cdot S_{av} )$ ...(1)

$R' = R \cdot S_{av} = BP_{av} / V_{av}$ ...(2)

In the above equations, $S_{av}$ represents an average cross-sectional area of the radial artery 22, and is treated as a constant. In the present embodiment, value R′ obtained by the equation (2), namely, by multiplying value R by value $S_{av}$ is handled as the peripheral resistance to be displayed by the display 50 (see the graph of Fig. 4). The denominator of the right side of the equation (1), namely, $V_{av} \cdot S_{av}$ represents an average flow amount of the blood flowing through the radial aretery 22. Since the average cross-sectional area of the arterial vessel is treated as a constant as indicated above, the peripheral resistance can be determined based on the blood flow amount in combination with the blood pressure. In the present invention, the ultrasonic wave generator and detector 38, 40, receiving circuit 44, step S5 stored in the ROM 46, the CPU 36 and the RAM 48 for effecting step S5, and others cooperate with each other to serve as the means for determining the blood flow rate regarding the arterial vessel extending through the peripheral portion of the subject, while step S6 stored in the ROM 46 and the CPU 36 and the RAM 48 for effecting step S6 serve as the means for calculating the peripheral resistance of the subject.

Step S6 is followed by step S7 at which the CPU 36 commands the display 50 to display the maximum, minimum and average blood pressure $BP_{max}$, $BP_{min}$, $BP_{av}$ and the peripheral resistance R′ in the two dimensional graph as shown in Fig. 4. Steps S1 through step S7 are repeated to display a time-wise varying trend of the blood pressure $BP_{max}$, $BP_{min}$, $BP_{av}$ and the peripheral resistance R′, on the display 50.

As is apparent from the foregoing, the present apparatus directly measures the peripheral resistance R′ based on the average blood pressure $BP_{av}$ and the average blood flow rate $V_{av}$ and displays the resistance R′, in contrast to the conventional indirect manner in which peripheral resistance is deduced indirectly through measurement of skin temperature. Thus, the present apparatus permits more reliable monitoring of dynamic condition of the circulatory organ of a subject, and more rapid detection of a state of shock of a subject, since the apparatus is capable of concurrently informing the operator or medical staff of the blood pressure and the directly measured, reliable peripheral resistance.

In the present apparatus, the ultrasonic wave generator and detector 38, 40 together with the pressure sensor 12 are disposed on the wrist band 10. Thus, both the blood pressure and the peripheral resistance are concurrently measured simply by applying the wrist band 10 to the wrist 20.

While in the illustrated embodiment the peripheral resistance R′ is calculated based on the average blood pressure $BP_{av}$ and the average blood flow rate $V_{av}$, it is possible to determine maximum and minimum blood flow rate $V_{max}$, $V_{min}$, and calculate maximum and minimum peripheral resistance $R'_{max}$ and $R'_{min}$ based on the maximum and minimum blood pressure $BP_{max}$, $BP_{min}$ and the determined maximum and minim blood flow rate $V_{max}$, $V_{min}$, respectively. In this case, it is possible to time-wise display all the maximum, minimum and average peripheral resistance Rmax, Rmin, R′ on the display 50.

The illustrated arrangement in which the pressure sensor 12 and the ultrasonic generator and detector 38, 40 are disposed on the wrist band 10, may be replaced with another arrangement in that the pair of ultrasonic wave generator and detector (38, 40) are disposed on the wrist band (20) and the pressure sensor (12) is disposed on an upper-arm band to be set around an upper arm of the subject so as to measure blood pressure based on pulse wave produced from a brachial artery extending through the upper arm. Since substantially the same blood pressure is measured at any location throughout a subject, the above-indicated another arrangement is permitted.

The means for determining blood pressure may be of a well-known type including an inflatable cuff to be wound around an upper arm of a subject.

While in the illustrated embodiment blood pressure and peripheral resistance are measured regarding a radial artery extending in the wrist 20, it is possible to make those measurements regarding an arterial vessel extending in a finger located more peripheral than the wrist, or regarding a dorsal pedal artery extending near an ankle of a foot.

As previously described, it is possible to determine peripheral resistance based on blood flow amount in combination with blood pressure, other than based on blood flow rate in combination with blood pressure. For example, blood flow amount is measured by an electromagnetic flow meter.

Although in the illustrated embodiment the blood flow rate is calculated by the receiving circuit 44, it is possible to adapt the CPU 36 to directly process signal SF supplied from the ultrasonic wave detector 40, according to software pre-stored in the ROM 46. In this case, the receiving circuit 44 is omitted.

While in the illustrated embodiment maximum, minimum and average blood pressure and peripheral resistance are determined based on pulse wave signal SM and flow rate signal SL each corresponding to one pulse of the puse wave, it is possble to determine a plurality of blood pressures and peripheral resistances based on signals SM and SL correspoding to a plurality of the lastly read pulses of the pulse wave, and calculate and display moving averages of the plurality of blood pressures and peripheral resistances. In this case, it is preferred that the apparatus employ a ring buffer for storing signals SM, SL corresponding to the lastly read pulses. For example, the CPU 36 or RAM 48 may be adapted to serve as the ring buffer.

The pressure sensor for detecting pulse wave from an arterial vessel extending through a peripheral portion of a subject, may be of a type having a pressing surface to be pressed against the arterial vessel via a body surface of the peripheral portion, and supporting at the pressing surface a pressure detector for detecting the pulse wave produced from the arterial vessel.

While in the illustrated embodiment the pressure transducer 18 is disposed in the air-tight space 16, it is possible to dispose the transducer 18 in the piping 24.

Although in the illustrated embodiment the ultrasonic wave generator 38 continuously generates ultrasonic wave with a predetermined frequency, toward the radial artery 22, it is possible to replace the generator 38 with a genertor of a type adapted to generate pulses of ultrasonic wave toward the artery so that the reflected pulses are utilized to determine blood flow rate regarding the artery.

While in the illustrated embodiment the display 50 timewise displays the peripheral resistance of the subject, it is possible to trasnmit signal representing the peripheral resistance, to an external monitoring device adapted to obtain from the same subject information other than the peripheral resistance.

Referring next to Fig. 5, there is shown a modified from of the peripheral resistance measuring apparatus of the invention. The same reference numerals as used in the embodiment of Figs. 1 and 2 are used to designate corresponding elements or parts of the embodiment of Fig. 5, and repetitive description thereabout will be omitted.

The present apparatus further serves as an oxymeter for measuring oxygen saturation of a subject. The apparatus includes a first and a second light emitter 54, 56 and a light detector 58. The first light emitter 54 emits a red light beam having a wavelength of about 660 mμ, while the second light -emitter 56 emits an infrared light beam having a wavelength of about 804 mμ. The first and second light emitter 54, 56 alternately emit the red and infrared light beams toward capillaries in skin (body surface) 19 of the subject as a result of being activated by drive signals SD3 and SD4, respectively, supplied thereto from the output interface 52. The light beams reflected from the skin are detected by the light detector 58. The light detector 58 generates electric signal SV representing intensity of the detected light beams, to a pulse wave detecting circuit 60. The pulse wave detecting circuit 60 separates, from signal SV, pulse wave singnal $SM_R$ when the red light beam is detected and pulse wave signal $SM_{IR}$ when the infrared light beam is detected, each of signals $SM_R$, $SM_{IR}$ representing pulse wave produced from the radial artery 22 synchronously heartbeat of the subject. Signals $SM_R$, $SM_{IR}$ are supplied to the CPU 36 via an A/D converter 62. The CPU 36 determines oxygen saturation of the blood flowing through the capillaries in the skin of the subject, based on signals $SM_R$, $SM_{IR}$ received, and commands the display 50 to display the determined oxygen saturation. More specifically described, oxygen saturation is determined based on a ratio of an absorption coefficient of the red light beam regarding blood flowing through capillaries, to an absorption coefficient of the infrared light beam regarding the blood, according to a predetermined relationship between the oxygen saturation and that ratio, and the ratio in question is determined based on a ratio of an amplitude of signal $SM_R$ to an amplitude of signal $SM_{IR}$, according to a predetermined relationship between the two ratios.

While the present invention has been described in its presently preferred embodiment and one modified form thereof with detailed particularities, it is to be understood that the invention may be embodied with other changes, modifications and improvements that may occur to those skilled in the art without departing from the scope and spirit of the invention defined in the appended claims.

**Claims**

1. An apparatus for detecting a resistance opposing blood flow through a peripheral portion (20) of a subject's body, comprising:
first determining means (12, 36) for determining blood pressure of said subject;
second determining means (38, 40, 44, 36) for determining at least one of a flow rate and a flow amount of blood which flows through an arterial vessel (22) extending through said peripheral portion (20) of said body; and
calculating means (36) for calculating said resistance based on said blood pressure and said at least one of the blood flow rate and the blood flow amount.

2. The apparatus as set forth in claim 1, wherein said second determining means comprises
an ultrasonic wave generator (38) for generating and transmitting ultrasonic wave toward said arterial vessel (22) extending through said peripheral portion (20) of said body, an ultrasonic wave detector (40) for detecting the ultrasonic wave reflected by said blood flowing through said arterial vessel, and
means (44, 36) for determining said blood flow rate based on the detected ultrasonic wave.

3. The apparatus as set forth in claim 2, wherein said first determining means comprises a pressure sensor (12) for detecting a pulse wave produced from said arterial vessel (22), said pressure
sensor being pressed against a body surface (19) of said subject just above said arterial vessel so as to detect said pulse wave, and
means (36) for determining said blood pressure based on magnitude of the detected pulse wave.

4. The apparatus as set forth in claim 3, further comprising
a wrist band (10) for supporting, on one (11) of opposite surfaces thereof, said ultrasonic wave generator and detector (38, 40) and said pressure sensor (12), said wrist band pressing, under said one of opposite surfaces thereof, said ultrasonic wave generator and detector and said pressure sensor against a radial artery (22) of said subject via said body surface (19) when said wrist band is placed around a wrist (20) of said subject.

5. The apparatus as set forth in claim 3 or claim 4, wherein said pressure sensor (12) comprises an elastic member (14) for defining a fluid-tight space (16), said elastic member being pressed against said arterial vessel (22) via said body surface (19) when pressure in said fluid-tight space is increased, and
a pressure transducer (18) disposed in said fluid-tight space, for detecting pressure variation in said fluid-tight space upon transmission of said pulse wave to said fluid-tight space from said arterial vessel.

6. The apparatus as set forth in claim 4 or claim 5, further comprising
third determining means (54, 56, 58, 60, 36) for determining oxygen saturation of blood which flows through capillaries in skin (19) of said subject, said third determining means comprising a first light emitter (54) for emitting toward said skin a light beam having a first wavelength, a second light emitter (56) for emitting toward said skin a light beam having a second wavelength different from said first wavelength, and a light detector (58) for detecting the light beams reflected from said skin, said wrist band (10) also supporting said first and second light emitters and said light detector, said third determining means further comprising means (60, 36) for determining said oxygen saturation based on the detected light beams.

7. The apparatus as set forth in any one of claims 1-6, wherein said second determining means (38, 40, 44, 36) determines at least one of an average flow rate and an average flow amount of said blood flowing through said arterial vessel (22).

8. The apparatus as set forth in any one of claims 1-7, further comprising
a display device (50) for displaying at least one value of maximum, minimum and average blood pressure; maximum, minimum and average blood flow rate; maximum, minimum and average resistance; and maximum, minimum and average value obtained by respectively multiplying said maximum, minimum and average resistance by an average sectional area of said arterial vessel (22).

9. The apparatus as set forth in claim 8, wherein said display device (50) displays said at least one value each time said pressure sensor (12) detects a pulse of said pulse wave from said arterial vessel (22).

10. The apparatus as set forth in any one of claims 1-9, wherein said second determining means (38, 40, 44, 36) determines said blood flow amount by multiplying said blood flow rate by an average sectional area of said arterial vessel (22).

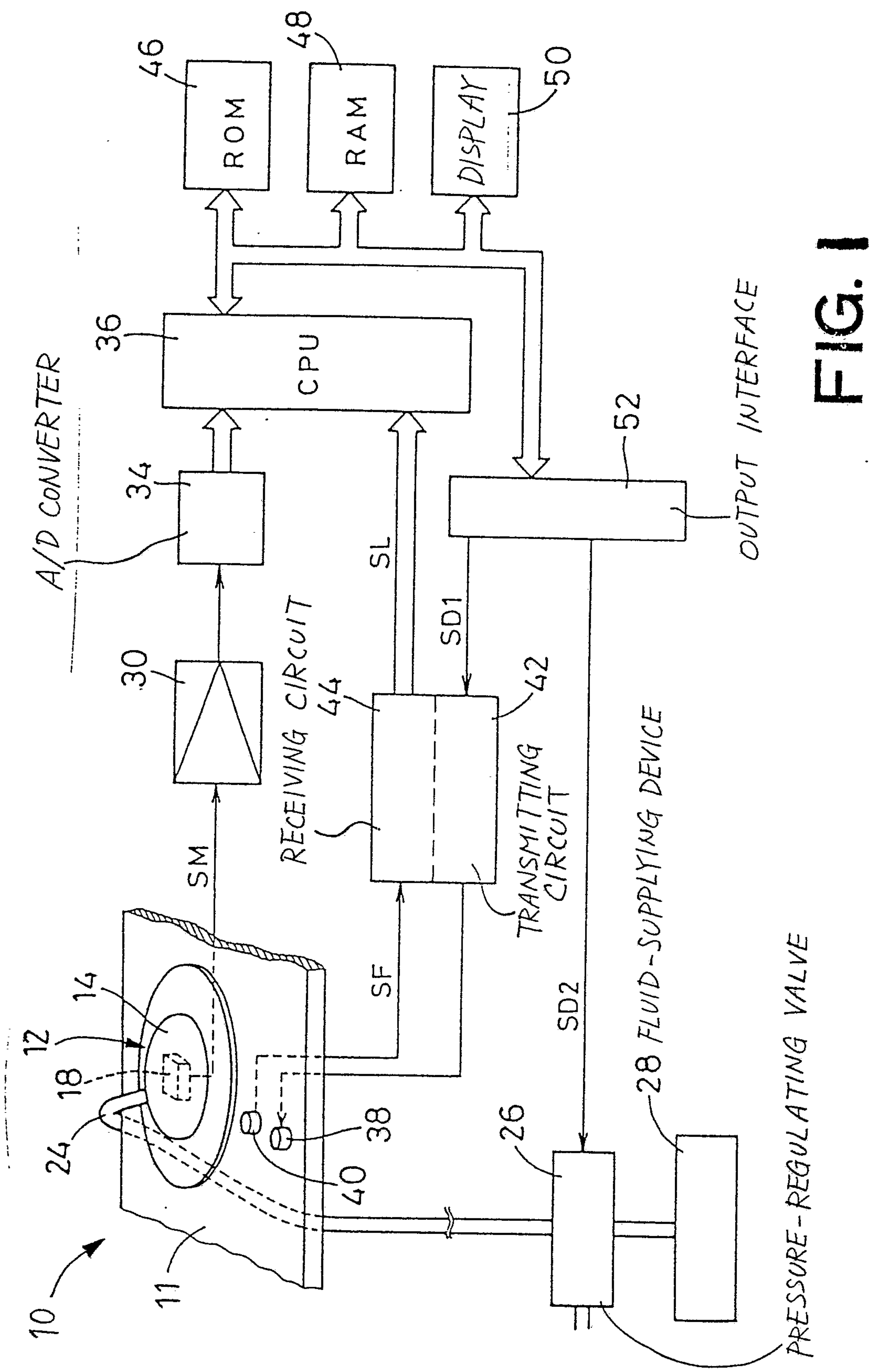
ROM
RAM
DISPLAY
CPU
A/D CONVERTER
RECEIVING CIRCUIT
TRANSMITTING CIRCUIT
OUTPUT INTERFACE
FLUID-SUPPLYING DEVICE
PRESSURE-REGULATING VALVE
SM
SL
SF
SD1
SD2
10
11
12
14
18
24
26
28
30
34
36
38
40
42
44
46
48
50
52

FIG. 1

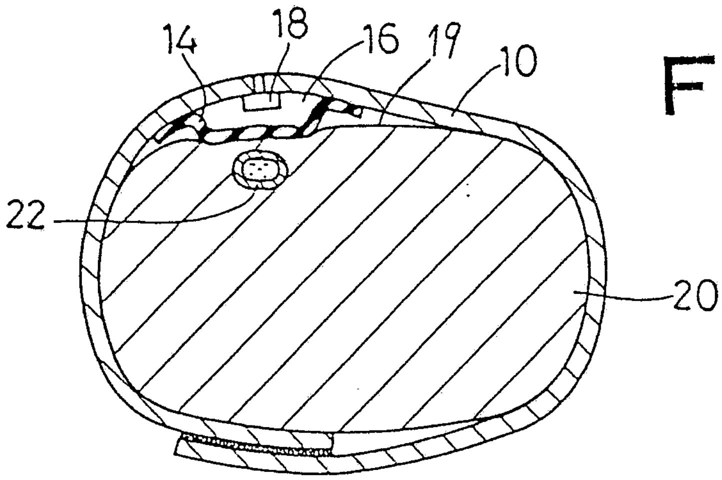
14
18
16
19
10
22
20

FIG.2

FIG.3

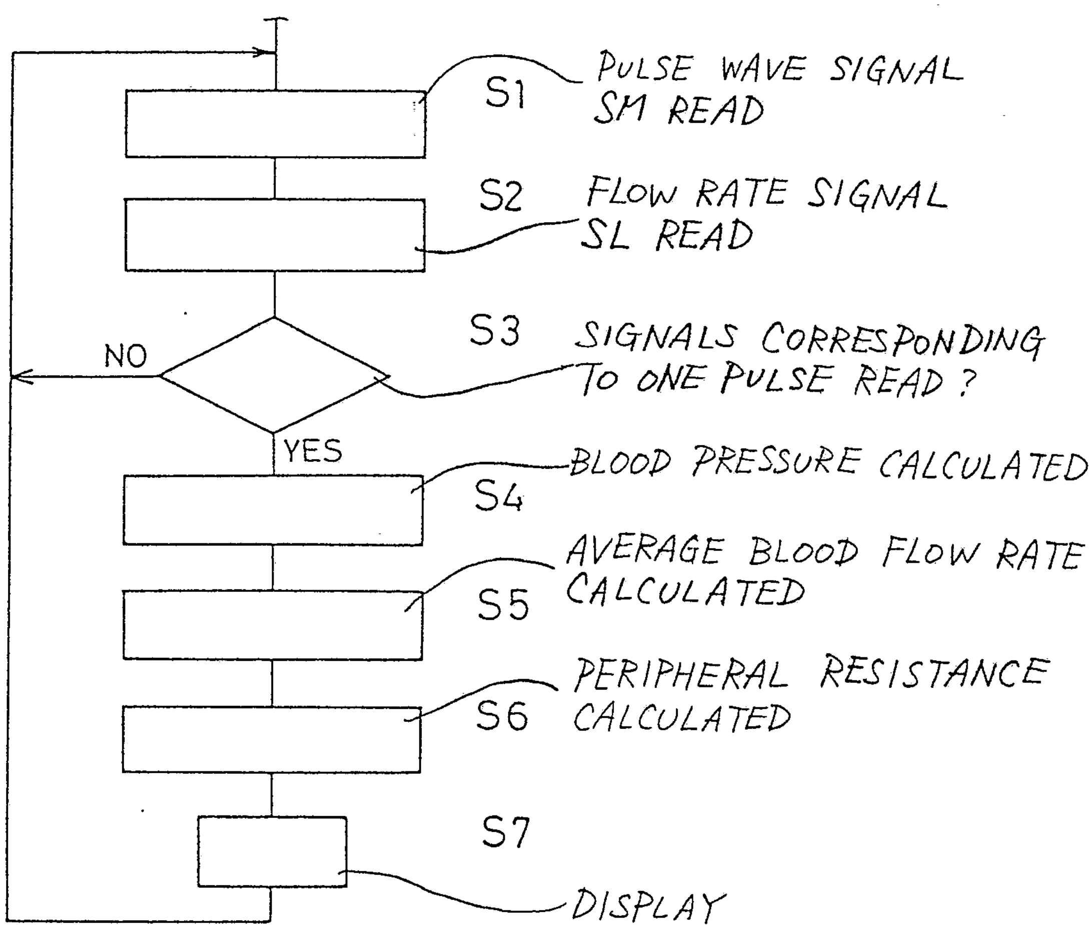
S1
PULSE WAVE SIGNAL SM READ
S2
FLOW RATE SIGNAL SL READ
S3
SIGNALS CORRESPONDING TO ONE PULSE READ ?
NO
YES
S4
BLOOD PRESSURE CALCULATED
S5
AVERAGE BLOOD FLOW RATE CALCULATED
S6
PERIPHERAL RESISTANCE CALCULATED
S7
DISPLAY

# FIG. 4

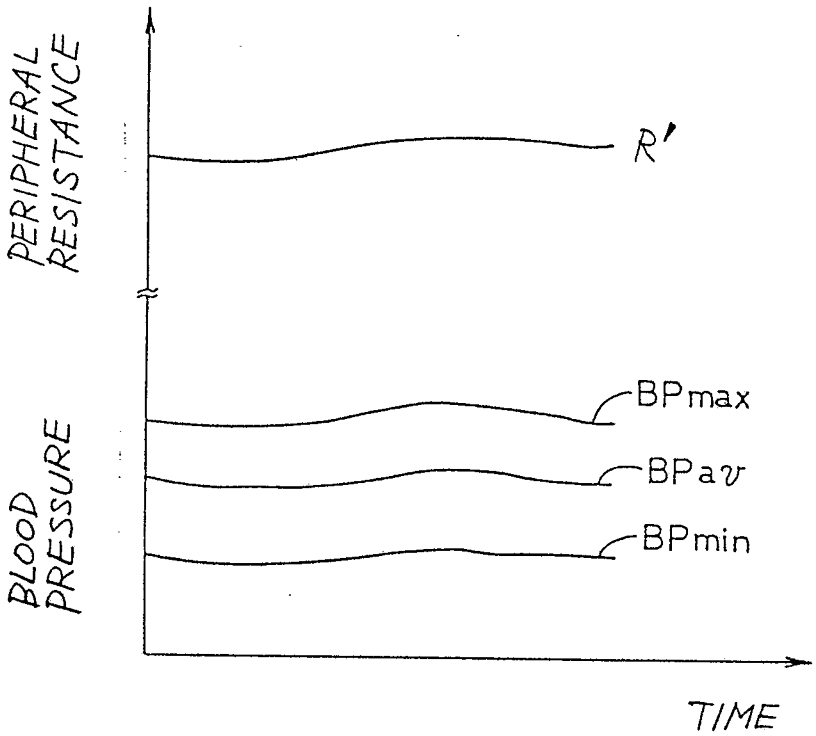
PERIPHERAL RESISTANCE
R'
BLOOD PRESSURE
BPmax
BPav
BPmin
TIME

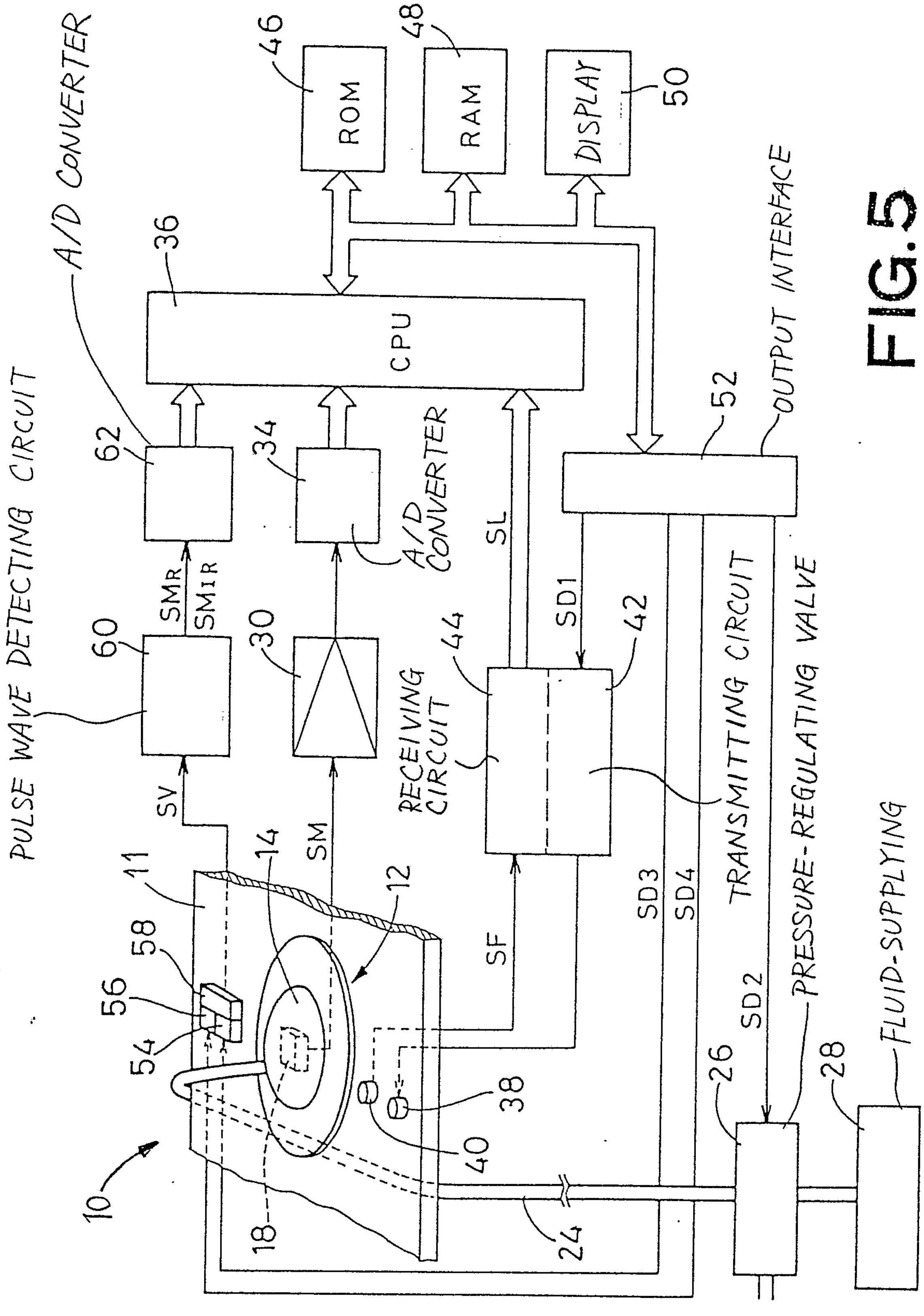
PULSE WAVE DETECTING CIRCUIT
A/D CONVERTER
ROM
RAM
DISPLAY
CPU
OUTPUT INTERFACE
A/D CONVERTER
RECEIVING CIRCUIT
TRANSMITTING CIRCUIT
PRESSURE-REGULATING VALVE
FLUID-SUPPLYING
SV
SM
SMR
SMIR
SL
SF
SD1
SD2
SD3
SD4
10
11
12
14
18
24
26
28
30
34
36
38
40
42
44
46
48
50
52
54
56
58
60
62

FIG.5

European Patent Office

# EUROPEAN SEARCH REPORT

Application Number

EP 89 30 1758

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| Y<br>A | US-A-4 703 758 (Y. OMURA)<br>* Abstract; column 3, lines 36-55; claims 1,6-9; figures 5-8 *<br>--- | 1,7<br>2,3 | A 61 B 5/02<br>A 61 B 8/06 |
| Y | MEDICAL AND BIOLOGICAL ENGINEERING, vol. 11, no. 6, November 1973, pages 678-690, London, GB; D.A. McDONALD et al.: "Left ventricular output derived from the time-derivative and phase velocities of the aortic pressure wave"<br>* Page 687, paragraph: "Nature of the input impedance" *<br>--- | 1,7 | |
| A | EP-A-0 227 119 (NIPPON COLIN CO., LTD)<br>* Abstract; figures 1,2 *<br>--- | 6,8 | |
| A | US-A-4 660 566 (Y. PALTI)<br>* Abstract; column 11, line 1 - column 12, line 10; figures 12-16 *<br>--- | 1-5,8 | TECHNICAL FIELDS SEARCHED (Int. Cl.4) |
| A | EP-A-0 035 325 (NATIONAL RESEARCH DEVELOPMENT CORP.)<br>* Abstract; figure 1 *<br>----- | 10 | A 61 B |

The present search report has been drawn up for all claims

| Place of search | Date of completion of the search | Examiner |
|---|---|---|
| THE HAGUE | 31-05-1989 | HUNT,B.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)